# EUROPEAN PATENT SPECIFICATION

(11) **EP 1 361 217 B1**
(45) Date of publication and mention of the grant of the patent: **23.03.2005**
(21) Application number: 03009516.0
(22) Date of filing: 22.01.2002
(51) Int. Cl.: C07D 233/42

(54) **4-Thionoimidazolidine derivatives as oil-soluble additives for lubricating oils**
4-Thionoimidazolidinderivate als öllösliche Additive für Schmieröle
Dérivés de 4-thionoimidazolidine, additifs oleosolubles pour huiles lubrifiantes

(30) Priority: 24.01.2001 US 263776 P
(43) Date of publication of application: 12.11.2003
(62) Divisional of application: 02250439.3
(73) Proprietor: Rohm and Haas Company, Philadelphia, Pennsylvania 19106-2399 (US)
(72) Inventor: Mukkamala, Ravindranath, Houston, Texas 77062 (US)
(74) Representative: Kent, Venetia Katherine

(56) References cited:
- GB-A- 1 053 716
- US-A- 4 189 587
- F.ASINGER, K.HENTSCHEL, A.SAUS: "Zum Substitutionverhalten von Imidazolidin-4-thionen" MONATSHEFTE FUR CHEMIE, 107, 35-41, (1976), XP001068694

## Description

### Background

This invention relates generally to heterocyclic compounds useful as ashless oil-soluble additives for lubricating oils.

Zinc dialkyldithiophosphates (ZDDP) are widely used as lubricant additives. The principal disadvantages of these compounds are that an ash residue is produced by the zinc as the additive is consumed, and that phosphorus is known to affect the efficiency of catalytic converters in motor vehicles, thereby causing emissions problems. An ashless, non-phosphorus alternative to ZDDP would be extremely useful.

Dithiohydantoin compounds are disclosed in European Patent Application No. EP 0 728 747 A1. However, the compounds are not within the scope of the present invention, and moreover, are disclosed only for pharmaceutical applications.

The problem addressed by this invention is to find improved phosphorus-free ashless oil-soluble additives for lubricating oils.

### Statement of Invention

The present invention is directed to a compound having the formula 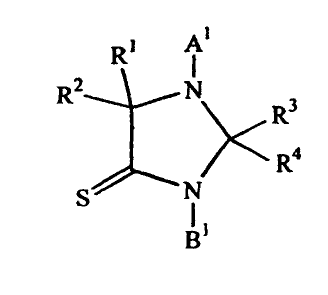 wherein A¹ and B¹ are independently hydrogen,

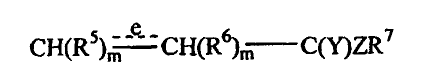

CH₂NHR⁸

or 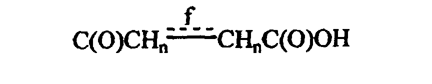 R¹, R², R³ and R⁴ are independently hydrogen, alkyl, alkenyl, aryl or aralkyl; or R¹ and R², or R³ and R⁴, combine with the carbon atom to which they are attached to form an alkyl or alkenyl ring; Y is O or S; Z is O, S or NR⁹; m is 0 when bond e is a double bond and 1 when e is a single bond; n is 1 when bond f is a double bond and 2 when f is a single bond; R⁵ is C(Y)ZR⁷, hydrogen or C₁-C₄ alkyl; R⁶ is hydrogen or C₁-C₄ alkyl; R⁷, R⁸ and R⁹ are independently hydrogen, alkyl, alkenyl, aryl or aralkyl;
provided that at least one of A¹ and B¹ is not hydrogen,
and wherein:
"alkyl" is a hydrocarbyl group having from 1 to 20 carbon atoms in a linear, branched or cyclic arrangement, and having from 0 to 2 oxygen, nitrogen or sulfur atoms, optionally substituted with one or more halo, hydroxy, alkoxy, alkanoyl or amido groups, and wherein said alkoxy, alkanoyl and amido groups are optionally substituted with one or more halo substituents; "alkenyl" is alkyl as defined above in which at least one single bond has been replaced with a double bond;
"aryl" is an aryl group is a substituent derived from an aromatic compound, including heterocyclic aromatic compounds having hetero atoms chosen from among oxygen, nitrogen and sulfur, having a total of from 5 to 20 ring atoms and having one or more rings which are separate or fused, wherein said aryl group is optionally substituted with one or more halo, alkyl, alkenyl, hydroxy, alkoxy, alkanoyl or amido group, and wherein said alkyl, alkenyl, alkoxy, alkanoyl or amido groups are optionally substituted with one or more halo groups; and "aralkyl" is an alkyl as defined above substituted by an aryl as defined above.

In another aspect, the present invention provides a composition comprising a lubricating oil and from 0.1% to 20% of the compound defined above. Preferably, the composition contains from 0.5% to 10% of said compound. In another aspect, the present invention provides the use of a compound as defined above in an amount of from 0.1 to 20% for improving the anti-wear and anti-corrosion characteristics of a lubricating oil.

### Detailed Description

An "alkyl" group is a hydrocarbyl group having from one to twenty carbon atoms in a linear, branched or cyclic arrangement, and having from 0 to 2 oxygen, nitrogen or sulfur atoms. Substitution on alkyl groups of one or more halo, hydroxy, alkoxy, alkanoyl or amido groups is permitted; alkoxy, alkanoyl and amido groups may in turn be substituted by one or more halo substituents. Preferably, alkyl groups contain from one to twelve carbon atoms and from 0 to 1 oxygen, nitrogen or sulfur atoms. An "alkenyl" group is an "alkyl" group in which at least one single bond has been replaced with a double bond. An "aryl" group is a substituent derived from an aromatic compound, including heterocyclic aromatic compounds having heteroatoms chosen from among nitrogen, oxygen and sulfur. An aryl group has a total of from five to twenty ring atoms, and has one or more rings which are separate or fused. Substitution on aryl groups of one or more halo, alkyl, alkenyl, hydroxy, alkoxy, alkanoyl or amido groups is permitted, with substitution by one or more halo groups being possible on alkyl, alkenyl, alkoxy, alkanoyl or amido groups. An "aralkyl" group is an "alkyl" group substituted by an "aryl" group.

In formula I, e and f indicate whether the bond between the adjacent carbons is a single or double bond, which is determined by the alkylating agent used to introduce the substituent, as described hereinbelow.

In one aspect of the invention, a tetraalkylimidazolidinethione (TAIT), or an imidazolidinethione having from one to three alkyl groups, is alkylated with an acrylate ester to produce a compound having a CHR⁵CHR⁶C(O)OR⁷ group, as shown below for R⁵= R⁶=H and R⁷=alkyl. If R¹, R², R³ and R⁴ are all methyl, the TAIT is known as TMIT. 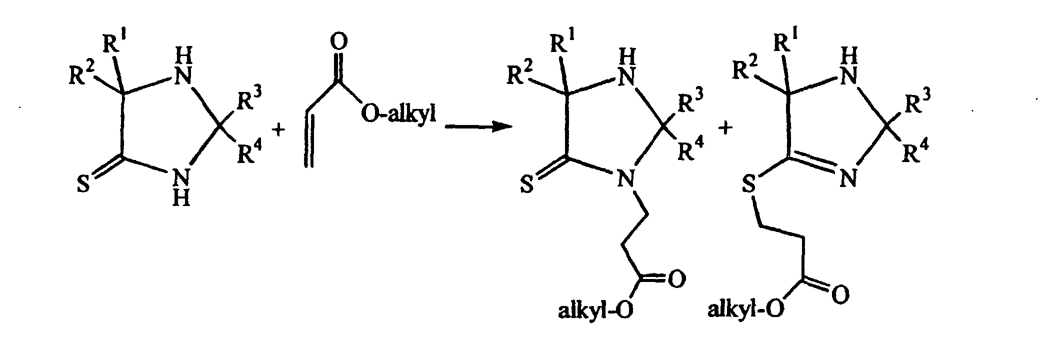 The extent of N-alkylation versus S-alkylation varies with the identity of the R groups on the imidazolidenethione ring and with the alkylating agent, as shown below in the Examples.

In another aspect of this invention, a TAIT or an imidazolidinethione having from one to three alkyl groups is alkylated with an alkyl propiolate to produce a compound in which the ester side chain has a carbon-carbon double bond. In another aspect of this invention, a TAIT or unsubstituted imidazolidinethione is alkylated with an imine, CH₂=NR⁸. In another aspect of this invention, a TAIT or an imidazolidinethione having from one to three alkyl groups is alkylated with maleic or succinic anhydride to produce a compound having a C(O)CH=CHC(O)OH or C(O)CH₂CH₂C(O)H side chain, respectively, with alkylation occurring mainly on the sulfur.

In one embodiment of the invention, the group ZR⁷ in a CHR⁵CHR⁶C(Y)ZR⁷ side chain or a CH=CHC(Y)ZR⁷ side chain contains a thioethyl group, i.e., a group having the structure -CH₂CH₂S-, where one of the CH₂ and the sulfur is attached to the C(Y) functionality and the other is attached to an alkyl, alkenyl or aralkyl group. For example, ZR⁷ can be OCH₂CH₂S-R, where R is alkyl, alkenyl or aralkyl; when Y is O, and R⁵ and R⁶ are H, the side chain is CH₂CH₂C(O)OCH₂CH₂S-R.

In a preferred embodiment of the invention, from 0.1 to 10% of a compound of formula I is added to a lubricating oil. More preferably, from 0.5 to 10% of a compound of formula I is added to a lubricating oil, and most preferably, from 0.5 to 3%. A lubricating oil is a natural or synthetic oil, having suitable viscosity for use as a lubricant, or a mixture thereof.

### Examples

### Example 1: Alkylation of Tetraalkylimidazolidinethiones with Alkyl Acrylates

TMIT was prepared according to the procedure given in U.S. Pat. No. 5,057,612, as follows.

To a mechanically-stirred mixture of ammonium sulfide (0.4 moles, 136 mL, 20 wt% aqueous solution), sodium cyanide (14.7 g, 0.3 moles), ammonium chloride (16.1 g, 0.3 moles) and water (80 mL), acetone (44 mL, 0.6 moles) was added drop-wise over a period of 30 min.; during the addition of acetone, the reaction temperature rose to about 36°C. The reaction mixture was then externally heated to 65°C for a period of 6-7 hours. The reaction mixture was cooled to 0-5°C using an ice bath, and the white solid was filtered, washed with cold water and suction-dried. The yield of TMIT was 44.6 grams (94 %); melting point: 155°C. IR: 3521, 2976, 1657, 1524, 1462 cm⁻¹; ¹H NMR (CDCl₃, 500 MHz): δ 1.46 (s, 6 H), 1.44 (s, 6 H) ppm; ¹³C NMR (CDCl₃, 125 MHz): δ 207.7, 78.4, 70.9, 29.9, 29.9 ppm.

7,14-diazadispiro[5.1.5.2]pentadecane-15-thione (DDPT), 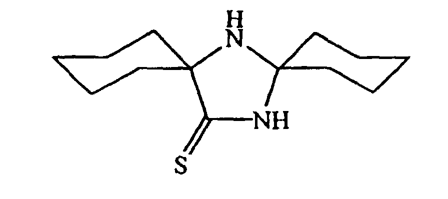 was prepared according to the procedure described for TMIT from ammonium sulfide (0.4 moles, 136 mL, 20 wt% aqueous solution), sodium cyanide (14.7 g, 0.3 moles), ammonium chloride (16.1 g, 0.3 moles) and water (80 mL); with addition of cyclohexanone (58.8 g, 0.6 moles). The product was obtained as a white solid (69.8 grams, 98%), and melted at 229°C. IR: 3127, 2925, 2855, 1516, 1454 cm⁻¹; ¹H NMR (CDCl₃, 500 MHz): δ 9.8 (bs, 1H), 1.9(dt, 2H), 1.8-1.2 (m, 18H) ppm; ¹³C NMR (CDCl₃, 125 MHz): δ 207.8, 81.0, 72.9, 39.6, 37.8, 24.9, 24.6, 23.0, 21.9 ppm.

Unless otherwise specified, tetraalkylimidazolidinethiones were allowed to react with alkyl acrylates in acetonitrile in the presence of 50 mole% of Cs₂CO₃ at room temperature for 10-15 hours (TMIT) or for 5 hours (DDPT) to produce compounds having the following structure: 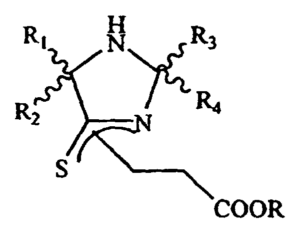 Detailed procedures and product analyses for several products are presented in Examples 2-8. The acrylates are abbreviated as follows: MA = methyl acrylate; 2-EHA = 2-ethylhexyl acrylate; LA = lauryl acrylate; BA = butyl acrylate; and TUA=3-thiaundecyl acrylate. Yield is given in %, the ratio of N-alkylated adduct to S-alkylated adduct (N/S) as a ratio of percentages or as "nd" (not determined), the physical state (state) as "L" (liquid), "SS" (soft solid) or "SG" (sticky gum), and the oil solubility (oil sol) as a weight percent. Oil solubility was measured at room temperature in EXCEL HC 100 lubricating base oil (available from Pennzoil Corp.). The adduct ratio, N/S, was determined from integration of proton NMR signals. The results for all acrylate adducts are presented below in Table 1.

### Example 2: Adduct of TMIT and 2-EHA

A mixture of TMIT (1.0 g, 6.33 mmol), 2-ethylhexyl acrylate (1.16 g, 6.33 mmol) and cesium carbonate (1.0 g, 3.3 mmol) in acetonitrile (15 mL) was stirred at room temperature for 24 h. The reaction mixture was filtered to separate solid cesium carbonate and solvent was evaporated from the filtrate to obtain the product as a colorless oil (1.9 g, 88 %). IR: 3325, 2961, 1732, 1595, 1480 cm⁻¹; ¹H NMR (CDCl₃, 500 MHz): δ 3.96 (overlapping d, 2 H), 3.83 (t, 1.72 H), 3.22 (t, 0.28 H), 2.82 (t, 1.72 H), 2.71 (t, 0.28 H), 1.91 (bs, 1H), 1.42 (s, 6 H), 1.40 (s, 6H), 1.35-1.20 (m, 8 H), 0.85 (overlapping t, 6 H) ppm; ¹³C NMR (CDCl₃, 125 MHz): δ 205.8, 173.6, 171.9, 171.3, 130.2, 128.5, 88.7, 82.9, 70.35, 69.6, 67.2, 66.9, 66.8, 40.6, 38.6, 33.9, 31.4, 30.26, 30.21, 28.79, 28.71, 28.23, 25.8, 23.64, 22.83, 13.9, 10.9 ppm.

### Example 3: Adduct of TMIT and LA

A procedure similar to that of Example 2 was used. Starting from TMIT (1.0 g, 6.33 mmol), lauryl acrylate (1.5 g, 6.33 mmol) and cesium carbonate (1.0 g, 3.3 mmol) in acetonitrile (15 mL), the product was isolated as a colorless oil (1.7 g, 68 %). IR: 3326, 2925, 1732 1596, 1480 cm⁻¹; ¹H NMR (CDCl₃, 500 MHz): δ 4.18 (overlapping d, 2H), 3.86 (t, 1.78 H), 3.36 (t, 0.22 H), 2.85 (t, 1.78 H), 2. 75 (t, 0.22 H), 1.90 (bs, 1H), 1.62 (m, 2H), 1.48 (s, 6H), 1.44 (s, 6H), 1.4-1.2 (m, 18 H), 0.88 (t, 3H) ppm.

### Example 4: Adduct of TMIT and BA

A procedure similar to that of Example 2 was used. Starting from TMIT (1.0 g, 6.33 mmol), n-butyl acrylate (0.81 g, 6.33 mmol) and cesium carbonate (1.0 g, 3.3 mmol) in acetonitrile (15 mL), the product was isolated as a colorless oil (1.3 g, 72 %). IR: 3323, 2961, 1732, 1582, 1483 cm⁻¹; ¹H NMR (CDCl₃, 500 MHz): δ 4.08 (t, 2H), 3.85 (t, 2H), 2.84 (t, 2H), 1.95 (bs, 1 H), 1.60 (m, 2H), 1.46 (s, 6H), 1.42 (s, 6H), 1.36 (m, 2H), 0.91 (t, 3H) ppm.

### Example 5: Adduct of DDPT and LA

A procedure similar to that of Example 2 was used. Starting from DDPT (1.0 g, 4.2 mmol), lauryl acrylate (1.0 g, 4.2 mmol) and cesium carbonate (0.68 g, 2.1 mmol) in acetonitrile (25 mL), the product was isolated as a light-yellow, low-melting solid (1.9 g, 95 %). IR: 2927, 2845, 1733, 1474 cm⁻¹; ¹H NMR (CDCl₃, 500 MHz): δ 4.15 (t, 2H), 3.85 (t, 2H), 2.85 (t, 2H), 2.03 (dt, 2H), 1.8-1.2 (m, 38 H), 0.88 (t, 3H) ppm.

### Example 6: Adduct of TAIT Mixture Prepared from Acetone/Methyl Isobutyl Ketone/Methyl Ethyl Ketone/Cyclohexanone and EHA

A TAIT mixture was prepared from an equimolar mixture of the four title ketones according to the procedure used for preparation of TMIT, using ammonium sulfide (136 mL, 0.4 moles, 20 wt% aqueous solution), sodium cyanide (14.7 g, 0.3 moles), ammonium chloride (16.1 g, 0.3 moles), water (80 mL), cyclohexanone (14.7 g, 0.15 moles), acetone (8.7 g, 0.15 moles) ethyl methyl ketone (10.8 g, 0.15 moles), and methyl isobutyl ketone (15.0 g, 0.15 moles) to obtain an oily layer at the end of the heating period. The oil layer was extracted into chloroform (350 mL), washed with water and dried with anhydrous potassium carbonate. Solvent evaporation yielded the product as a thick oil that slowly turned into a sticky gray solid (36 grams, yield: 55 % for an average molecular weight of 220). IR: 3361, 2962, 2874, 1605, 1520, 1459 cm⁻¹; ¹H NMR (CDCl₃, 500 MHz): δ 2.24 (d), 2.06 (s), 1.85-1.91 (m), 1.86-1.56 (m), 1.50-1.46 (m), 1.45-1.34 (m), 1.26-1.11 (bm), 1.39 (t), 0.99 (dd), 0.95-0.84 (m) ppm; ¹³C NMR (CDCl₃, 125 MHz): δ 207.8, 207.62, 207.60, 207.43, 207.40, 207.01, 206.89, 206.68, 206.66, 81.6, 81.18, 81.14, 80.70, 80.65, 78.38, 78.31, 73.95, 73.30, 72.82, 70.79, 70.46, 70.18 and several peaks between 40-10 ppm.

A procedure similar to that of Example 2 was used for the reaction with 2-EHA. Starting from the TAIT product described in the preceding paragraph (1.0 g, ca. 4.5 mmol), 2-ethylhexyl acrylate (0.82 g, 4.5 mmol) and cesium carbonate (0.75 g, 2.25 mmol) in acetonitrile (20 mL), the product was isolated as a yellow oil and solid mixture (1.8 g, 99 %). IR: 3325, 2933, 2860, 1732, 1480 cm⁻¹.

### Example 7: Adduct of TAIT Mixture Prepared from Methyl Ethyl Ketone and BA

A cis-trans TAIT mixture was obtained by applying the procedure used for preparation of TMIT to ammonium sulfide (136 mL, 0.4 moles, 20 wt% aqueous solution), sodium cyanide (14.7 g, 0.3 moles), ammonium chloride (16.1 g, 0.3 moles), water (80 mL), and ethyl methyl ketone (54.1 g, 0.75 moles) to obtain an oily layer at the end of the heating period. The oil layer was extracted into chloroform (350 mL), washed with water and dried with anhydrous potassium carbonate. Solvent evaporation yielded the product as a thick oil that turned into a sticky dirty-white solid. This solid was washed quickly with cold water and suction dried to give a white powder (23 g, yield: 41%) that melted at 72°C. IR: 3320, 3128, 2966, 1533, 1457, 1371 cm⁻¹; ¹H NMR (CDCl₃, 500 MHz): δ 1.85-1.65 (m, 4H), 1.44-1.36 (4s, 6H), 0.99-0.91 (m, 6H) ppm; ¹³C NMR (CDCl₃, 125 MHz): δ 207.15, 207.07, 81.24, 81.17, 73.69, 73.51, 35.49, 34.99, 33.85, 33.56, 28,56, 28.29, 27.82, 27.24, 8.55, 8.46, 8.25 ppm.

A procedure similar to that of Example 2 was used for the reaction with BA. Starting from the TAIT product described in the preceding paragraph (4.0 g, 21.5 mmol), n-butyl acrylate (2.8 g, 21.5 mmol) and cesium carbonate (3.5 g, 10.8 mmol) in acetonitrile (50 mL), the product was isolated as a yellow oil (6.1 g, 90%). IR: 3351, 2965, 2875, 1732, 1482 cm⁻¹; ¹H NMR (CDCl₃, 500 MHz): δ 4.05 (t, 2H), 3.95 (m), 3.80 (m), 3.63 (m), 2.95 (m), 2.82 (m), 2.67 (m), 1.80-1.51 (m, 6 H), 1.35 (m, 8H), 0.88 (m, 9H) ppm; ¹³C NMR (CDCl₃, 125 MHz): δ 205.31, 205.05, 171.2, 85.77, 85.67, 72.44, 72.21, 64.48, 40.28, 34.55, 33.93, 32.65, 33.63, 31.06, 31.03, 30.38, 28.61, 28.21, 26.46, 26.33, 18.91, 13.49 ppm.

### Example 8: Adduct of TMIT and 3-Thiaundecyl Acrylate

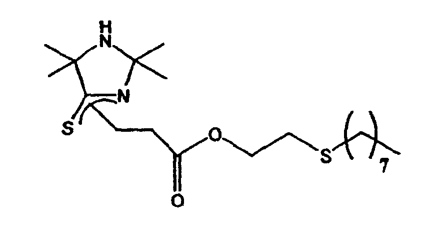

A procedure similar to that of Example 2 was used. Starting from TMIT (1.0 g, 6.33 mmol), 3-thiaundecyl acrylate (1.4 g, 6.33 mmol) and cesium carbonate (1.0 g, 3.3 mmol) in acetonitrile (20 mL), the product was isolated as a light yellow oil (2.0 g, 83 %). IR: 2961, 1734,1481 cm⁻¹; ¹H NMR (CDCl₃, 500 MHz): δ 4.22 (t, 2H), 3.84 (t, 2H), 2.84 (t, 2H), 2.71 (t, 2H), 2.52 (t, 2H), 1.55 (m, 2H), 1.46 (s, 6H), 1.42 (s, 6H), 1.4-1.2 (m, 10H), 0.85 (t, 3H) ppm; ¹³C NMR (CDCl₃, 125 MHz): δ 205.9, 170.9, 82.9, 69.5, 63.7, 40.4, 32.2, 31.6, 31.3, 30.2, 30.1, 29.5, 29.0, 28.6, 28.2, 22.4, 13.9 ppm.

**Table 1:**

| TAIT-Acrylate Ester Addition Products and Oil Solubilities | | | | | | |
|---|---|---|---|---|---|---|
| Ex. No. | TAIT | acrylate | yield | N/S | state | oil sol |
| | TMIT | MA | 85 | 83/17 | L | <2 |
| 2 | TMIT | 2-EHA | 88 | 86/14 | L | >20^{a} |
| 3 | TMIT | LA | 68 | 89/11 | L | >20^{a} |
| 4 | TMIT | BA | 72 | >97/<3 | L | <5 |
| | DDPT | MA | 20 | >99/<1 | SS | <5 |
| | DDPT | BA | 96 | >99/<1 | SS | <5 |
| | DDPT | 2-EHA | 95 | >99/<1 | SG | ca. 5^{b} |
| 5 | DDPT | LA | 95 | ca. 95/5 | SG | ca. 10^{b} |
| | mixture^{c} | 2-EHA | 89 | nd | L | ca. 10 |
| 6 | mixture^{d} | 2-EHA | 99 | nd | L | ca. 10 |
| | mixture^{d} | LA | 99 | nd | L/S | ca. 10 |
| 7 | mixture^{e} | BA | 90 | nd | L | >10 |
| 8 | TMIT | TUA | 83 | >95/<5 | L | ca. 5 |

| | | | | | | |
|---|---|---|---|---|---|---|
| a. Miscible at room temperature to give a single clear phase. | | | | | | |
| b. The mixture with oil was an unclear dispersion, with the product from lauryl acrylate being more clear than that from 2-ethylhexyl acrylate. | | | | | | |
| c. TAIT produced from equimolar mixture of acetone/methyl isobutyl ketone/cyclohexanone. | | | | | | |
| d. TAIT produced from equimolar mixture of acetone/methyl isobutyl ketone/methyl ethyl ketone/cyclohexanone. | | | | | | |
| e. TAIT cis/trans mixture produced from methyl ethyl ketone. | | | | | | |

### Example 9: Efficacy Testing and Performance

Efficacy of four oil formulations was tested, including a base oil and one containing a commercial anti-wear ZDDP-based additive, ELCO-103. The samples tested were as follows: (1) EXCEL HC 100 base oil; (2) EXCEL HC 100 with 1% ELCO 103; (3) EXCEL HC 100 with 1% of the adduct of 2-EHA and TMIT (see Example 2); and (4) EXCEL HC 100 with 1% of an imine adduct of TMIT (see Example 15). Details of the tests are as follows:

4-Ball anti-wear test (ASTM D-4172). Load: 40 Kg; Temp: 75°C; Rotation rate: 1200 rpm; Time: 1 hour; Measured parameter: wear scar diameter in mm on the steel balls. The smaller the scar diameter, the more effective a given anti-wear additive.

Load carrying capacity (EP test, ASTM D-2783). Similar to the anti-wear test above, but starts at room temperature and the load on four rotating balls is constantly increased until the balls weld to each other. The quantities measured to assess performance are weld point load (kgf), scar diameter (mm at 100 kgf or 126 kgf) just before weld point, and load wear index (LWI) (average of sum of the corrected loads determined for 10 applied loads preceding the weld point, kgf). A higher LWI is an indication of better anti-wear properties.

Copper corrosion test (ASTM D-130). Copper metal specimens are immersed in the oil sample at 212°F (100°C) for three hours, and the appearance is then rated based on the tarnish acquired. Here, a lower rating reflects lesser corrosivity. For example, a rating of "1" indicates only a slight tarnish, with "1A" being a light orange and "1B" a dark orange; "2" would indicate moderate tarnish, with ratings of "A" through "E" indicating progressively darker colors.

Results of the tests are presented below in Table 2.

**Table 2:**

| Test Results for Lubricating Oils | | | | |
|---|---|---|---|---|
| sample | ASTM D-4172 scar diameter | ASTM D-130 corrosion | EP Test | |
| | | | scar diameter^{b} | LWI |
| 1 | 0.84 | 1B^{a} | 2.99@100 | 10.8 |
| 2 | 0.64 | 1A^{a} | 2.1@100 | 21.5 |
| 3 | 0.63 | 1A^{a} | 2.45@100 | 14.5 |
| 4 | 0.65 | 1A^{a} | 2.47@126 | 18.8 |

| | | | | |
|---|---|---|---|---|
| a. Each sample had a slight tarnish. | | | | |
| b. The actual weld point of samples 1-3 was 126 kgf, and that of sample 4 was 160 kgf. | | | | |

### Reference Example 10: Adduct of TMIT and Methyl Iodide

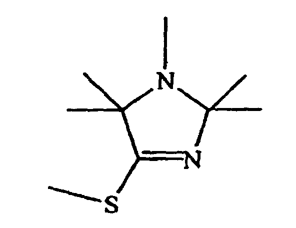

A mixture of TMIT (2.0 g, 12.6 mmol), methyl iodide (5.6 g, 40 mmol), and anhydrous potassium carbonate (8.3 g, 138. 2 mmol) in chloroform (45 mL) were stirred at room temperature for 2 days. The mixture was filtered, and solvent was evaporated to obtain the product depicted above as a liquid (1.9 g, 83%). ¹H NMR (CDCl₃, 500 MHz): δ 2.38 (s, 3H), 2.26 (s, 3H), 1.25 (s, 6H), 1.16 (s, 6H) ppm; ¹³C NMR (CDCl₃, 125 MHz): δ 175.1, 88.9, 70.3, 27.5, 26.4, 24.6, 12.6 ppm. The product was soluble in EXCEL HC 100 lubricating base oil only in an amount below 1% by weight.

### Reference Example 11: Adduct of DDPT and Methyl Iodide

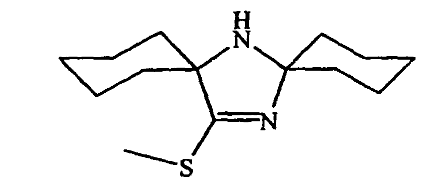

A mixture of DDPT (3.0 g, 12.6 mmol), methyl iodide (5.6 g, 40 mmol), and anhydrous potassium carbonate (8.3 g, 138. 2 mmol) in chloroform (30 mL) was stirred at room temperature for 2 days. The mixture was filtered, and solvent was evaporated to obtain the product as a thick liquid (2.45 g, 77%) that slowly turned into a soft solid. ¹H NMR (CDCl₃, 500 MHz): δ 2.41 (s, 3H), 1.75-1.1 (m, 20 H) ppm; ¹³C NMR (CDCl₃, 125 MHz): δ 174.9, 90.5, 72.6, 40.3, 37.5, 25.4, 25.2, 23.4, 22.4, 13.5 ppm. The product was soluble in EXCEL HC 100 at 5 weight % at room temperature, and remained clear at room temperature after 1 week.

### Reference Example 12: Adduct of TMIT and Methyl Propiolate

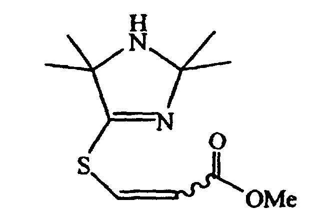

A mixture of TMIT (1.0 g, 6.33 mmol) and methyl propiolate (0.53 g, 6.33 mmol) in chloroform (15 mL) was strirred at room temperature for 24h, followed by heating at 45°C for another 24h. Solvent evaporation yielded the product depicted above as a light-yellow, crystalline solid (1.4 g, 92%). IR: 3329, 2974, 1706, 1606, 1436 cm⁻¹; ¹H NMR (CDCl₃, 500 MHz): δ 8.29 (d, J=16.0 Hz, 0.12 H), 8.15 (d, J=10 Hz, 0.88 H), 6.16 (d, J=16 Hz, 0.12 H), 6.19 (d, J=10 Hz, 0.88 H), 3.7 (s, 3H), 1.47 (s, 0.72 H), 1.45 (s, 0.72 H), 1.42 (s, 5.3 H), 1.36 (s, 5.3 H) ppm; ¹³C NMR (CDCl₃, 125 MHz): δ 171.4, 169. 6, 166.9, 141.3, 140.6, 118.9, 115.5, 114.1, 51.6, 30.5, 30.07, 30.06, 28.3 ppm. The product is an 85/15 mixture of cis/trans isomers. The product was soluble in EXCEL HC 100 at 10 weight % at 100°C, but precipitated at room temperature after 30 minutes.

### Reference Example 13: Adduct of Example 12 Product with Methyl Propiolate

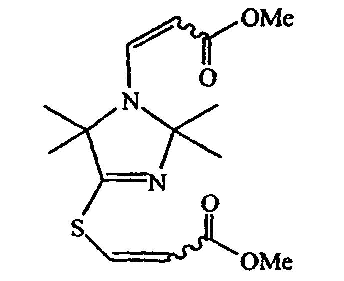

A mixture of the product made in Example 12 (0.1 g, 0.6 mmol) and methyl propiolate (0.053 g, 0.6 mmol) in deuterated chloroform (CDCl₃, 1 mL) was left at room temperature 3 days and then heated for 40h at 45°C. Solvent evaporation yielded RM-297 as a light-yellow, crystalline solid (1.5 g, 99%). IR: 2974, 1701, 1683, 1617, 1602, 1454 cm⁻¹; ¹H NMR (CDCl₃, 500 MHz): δ 8.29 (d, J=16.0 Hz), 8.08 (d, J=10 Hz), 8.075 (d, J=10 Hz), 7.36 (d, J=15 Hz), 6.09 (d, J=16 Hz), 6.08 (d, J=10 Hz), 6.03 (d, J=10 Hz), 4.73 (d, J=15 Hz), 3.71-3.69 (4 s, -CH₃), 1.48, 1.44, 1.34, 1.29 (4 s, - CH₃) ppm. The cis/trans ratio in the side chain attached to sulfur is 85/15, and in that attached to nitrogen it is 5/95. The product was soluble in EXCEL HC 100 at less than 5 weight % at 100°C.

### Reference Example 14: Adduct of DDPT with Methyl Propiolate

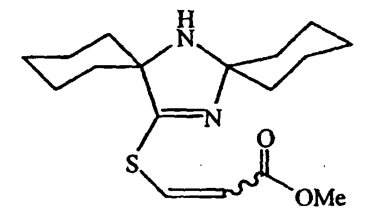

A mixture of DDPT (0.06 g, 0.252 mmol) and methyl propiolate (0.063 g, 0.75 mmol) in deuterated chloroform (CDCl₃, 1 mL)was heated at 45°C for 24h followed by standing at room temperature for 3 days. Solvent evaporation yielded the product depicted above as a light-yellow, crystalline solid (0.08 g, 99%). IR: 2932, 2854,1716, 1603, 1448 cm⁻¹; ¹H NMR (CDCl₃, 500 MHz): δ 8.35 (d, J=15.1 Hz, 0.4 H), 8.25 (d, J=10 Hz, 0.6 H), 6.15 (d, J=15.1 Hz, 0.4 H), 6.07 (d, J=10 Hz, 0.6 H), 3.71 (s, -CH₃), 3.75 (s, -CH₃), 3.72 (s, -CH₃), 1.9-1.1 (m, 20 H) ppm; ¹³C NMR (CDCl₃, 125 MHz): δ 170.9, 169.3, 166.9, 165.3, 153.0, 141.6, 141.0, 118.4, 115.1, 91.5, 91.0, 74.9, 74.3, 72.5, 72.5, 52.9, 51.6, 51.5, 36.7, 36.6, 25.4, 25.3, 25.0, 23.4, 23.3, 22.14, 22.12 ppm. The cis/trans ratio was 60/40. The product was soluble in EXCEL HC 100 at 5 weight % at 40°C; ca. 10 weight % at 100°C. A small amount of solid precipitated after 30 minutes.

### Example 15: Adduct of TMIT and an Imine Mixture

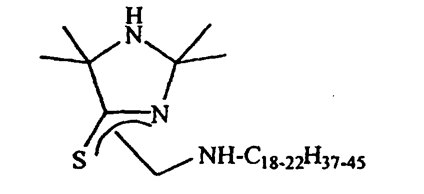

A mixture of TMIT (0.5 g, 3.16 mmol) and the formaldehyde imine (1.17 g, 3.2 mmol) of a mixture of branched C₁₈-C₂₂ primary amines (mixture of amines available from Rohm and Haas Co. under the name PRIMENE® JM-T) was heated in a sample vial at 120°C for 1h and the obtained liquid was cooled to room temperature yielding a thick syrup. IR: 3302, 1672, 1481, 1465, 1377 cm⁻¹; ¹H NMR (CDCl₃, 500 MHz): δ 5.4 (bm), 5.1 (s), 4.45-4.33 (5 s), 1.56-0.81 (3 m) ppm; ¹³C NMR (CDCl₃, 125 MHz): δ 208.4, 208.0, 206.5, 82.78, 82.42, 78.05, 70.88, 69.58, 69.42, 69.27, 68.35, 54.95, and several peaks at 40-14 ppm. The product was soluble in EXCEL HC 100 at 10 weight % at 100°C; at room temperature, 5% of the solid precipitated overnight.

## Claims

1. A compound having the formula 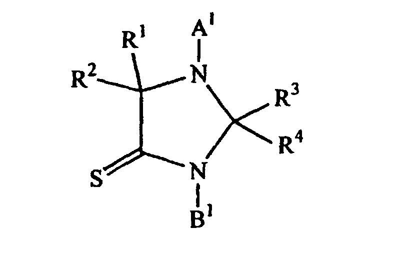 wherein A¹ and B¹ are independently hydrogen, 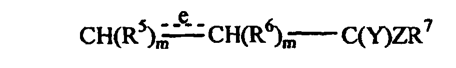
CH₂NHR⁸
or 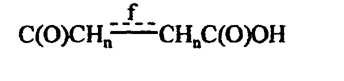 R¹, R², R³ and R⁴ are independently hydrogen, alkyl, alkenyl, aryl or aralkyl; or R¹ and R², or R³ and R⁴, combine with the carbon atom to which they are attached to form an alkyl or alkenyl ring; Y is O or S; Z is O, S or NR⁹; m is 0 when bond e is a double bond and 1 when e is a single bond; n is 1 when bond f is a double bond and 2 when f is a single bond; R⁵ is C(Y)ZR⁷, hydrogen or C₁-C₄ alkyl; R⁶ is hydrogen or C₁-C₄ alkyl; R⁷, R⁸ and R⁹ are independently hydrogen, alkyl, alkenyl, aryl or aralkyl;
provided that at least one of A¹ and B¹ is not hydrogen,
and wherein:
"alkyl" is a hydrocarbyl group having from 1 to 20 carbon atoms in a linear, branched or cyclic arrangement, and having from 0 to 2 oxygen, nitrogen or sulfur atoms, optionally substituted with one or more halo, hydroxy, alkoxy, alkanoyl or amido groups, and
wherein said alkoxy, alkanoyl and amido groups are optionally substituted with one or more halo substituents;
"alkenyl" is alkyl as defined above in which at least one single bond has been replaced with a double bond;
"aryl" is an aryl group is a substituent derived from an aromatic compound, including heterocyclic aromatic compounds having hetero atoms chosen from among oxygen, nitrogen and sulfur, having a total of from 5 to 20 ring atoms and having one or more rings which are separate or fused, wherein said aryl group is optionally substituted with one or more halo, alkyl, alkenyl, hydroxy, alkoxy, alkanoyl or amido group, and wherein said alkyl, alkenyl, alkoxy, alkanoyl or amido groups are optionally substituted with one or more halo groups; and
"aralkyl" is an alkyl as defined above substituted by an aryl as defined above.

2. A composition comprising a lubricating oil and from 0.1% to 20% of a compound as claimed in claim 1.

3. The composition of claim 2 which contains from 0.5% to 10% of a compound as claimed in claim 1.

4. The use of a compound as claimed in claim 1 in an amount of from 0.1 to 20% for improving the anti-wear and anti-corrosion characteristics of a lubricating oil.

## Patentansprüche

1. Verbindung mit der Formel 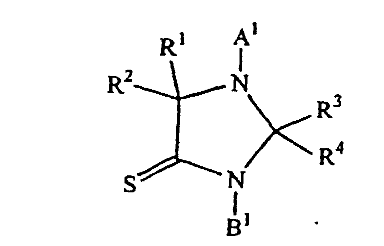 worin A¹ und B¹unabhängig voneinander Wasserstoff, 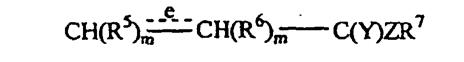
CH₂NHR⁸
oder 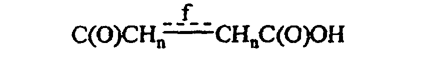 sind, R¹, R², R³ und R⁴ unabhängig voneinander Wasserstoff, Alky, Alkenyl, Aryl oder Aralkyl sind; oder R¹ und R² oder R³ und R⁴ mit dem Kohlenstoffatom, an welches sie gebunden sind, unter Bildung eines Alkyl- oder Alkenylringes kombinieren; Y O oder S ist; Z O, S oder NR⁹ ist, m 0 ist, wenn die Bindung e eine Doppelbindung ist, und 1 ist, wenn e eine Einfachbindung ist, n 1 ist, wenn die Bindung f eine Doppelbindung ist, und 2 ist, wenn f eine Einfachbindung ist, R⁵ C(Y)ZR⁷, Wasserstoff oder C₁-C₄ Alkyl ist, R⁶ Wasserstoff oder C₁-C₄ Alkyl ist, R⁷, R⁸ und R⁹ unabhängig voneinander Wasserstoff, Alkyl, Alkenyl, Aryl oder Aralkyl sind,
mit der Maßgabe, daß mindestens eines von A¹ und B¹ nicht Wasserstoff ist,
und wobei:
"Alkyl" ein Kohlenwasserstoffrest mit 1 bis 20 Kohlenstoffatomen in einer linearen, verzweigtkettigen oder cyclischen Anordnung ist und von 0 bis 2 Sauerstoff-, Stickstoff- oder Schwefelatome aufweist, gegebenenfalls substituiert mit einem oder mehreren Halogen-, Hydroxy-, Alkoxy-, Alkanoyl- oder Amidogruppen, und wobei die Alkoxy-, Alkanoyl- und Amidogruppen gegebenenfalls mit ein oder mehreren Halogensubstituenten substituiert sind,
"Alkenyl" ein Alkyl wie vorstehend definiert ist, worin mindestens eine Einfachbindung durch eine Doppelbindung ersetzt worden ist;
"Aryl" ein Arylrest ist, welcher ein von einer aromatischen Verbindung abgeleiteter Substituent ist, einschließlich heterocyclischen aromatischen Verbindungen mit Heteroatomen, ausgewählt aus Sauerstoff, Stickstoff und Schwefel, mit einer Gesamtheit von 5 bis 20 Ringatomen und mit einem oder mehreren Ringen, welche getrennt oder annelliert sind, wobei der Alkylrest gegebenenfalls mit ein oder mehreren Halogen-, Alkyl-, Alkenyl-, Hydroxy-, Alkoxy-, Alkanoyl- oder Amidogruppen substituiert ist, und wobei die Alkyl-, Alkenyl-, Alkoxy-, Alkanoyl- oder Amidogruppen gegebenenfalls mit einer oder mehreren Halogengruppen substituiert sind, und
"Aralkyl" ein Alkyl wie vorstehend definiert, substituiert mit einem Aryl wie vorstehend definiert, ist.

2. Zusammensetzung, umfassend ein Schmieröl und von 0,1% bis 20% einer Verbindung nach Anspruch 1.

3. Zusammensetzung nach Anspruch 2, welche von 0,5% bis 10% einer Verbindung, wie in Anspruch 1 beansprucht, enthält.

4. Verwendung einer Verbindung nach Anspruch 1 in einer Menge von 0,1 bis 20% zur Verbesserung der Antiverschleiß- und Antikorrosionseigenschaften eines Schmieröls.

## Revendications

1. Composé ayant la formule 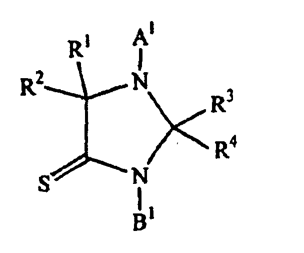 dans laquelle A¹ et B¹ représentent chacun indépendamment de l'autre un atome d'hydrogène, 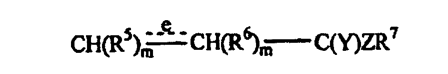
**CN**_{**2**}**NHR**^{**8**}
ou 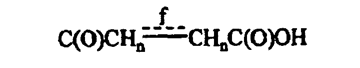 R¹, R², R³ et R⁴ représentent chacun indépendamment des autres un atome d'hydrogène ou un groupe alkyle, alcényle, aryle ou aralkyle ; ou R¹ et R², ou R³ et R⁴, se combinent à l'atome de carbone auquel ils sont liés pour former un noyau alkyle ou alcényle ; Y est O ou S ; Z est O, S ou NR⁹ ; m vaut 0 quand la liaison e est une double liaison et 1 quand e est une liaison simple ; n vaut 1 quand la liaison f est une double liaison et 2 quand f est une liaison simple ; R⁵ est C(Y)ZR⁷, un atome d'hydrogène ou un groupe alkyle en C₁-C₄ ; R⁶ est un atome d'hydrogène ou un groupe alkyle en C₁-C₄ ; R⁷, R⁸ et R⁹ représentent chacun indépendamment des autres un atome d'hydrogène ou un groupe alkyle, alcényle, aryle ou aralkyle ;
à la condition qu'au moins l'un de A¹ et B¹ ne soit pas un atome d'hydrogène,
et où :
"alkyle" est un groupe hydrocarbyle ayant de 1 à 20 atomes de carbone selon un arrangement linéaire, ramifié ou cyclique, et ayant de 0 à 2 atomes d'oxygène, d'azote ou de soufre, éventuellement substitué par un ou plusieurs groupes halogéno, hydroxy, alcoxy, alcanoyle ou amido, et lesdits groupes alcoxy, alcanoyle et amido étant éventuellement substitués par un ou plusieurs substituants halogéno ;
"alcényle" représente un groupe alkyle tel que défini ci-dessus, dans lequel au moins une liaison simple a été remplacée par une double liaison ;
"aryle" désigne un groupe aryle et est un substituant qui dérive d'un composé aromatique, y compris les composés aromatiques hétérocycliques ayant des hétéroatomes choisis parmi l'oxygène, l'azote et le soufre, ayant en tout de 5 à 20 atomes nucléaires et ayant un ou plusieurs noyaux qui sont séparés ou condensés, ledit groupe aryle étant éventuellement substitué par un ou plusieurs groupes halogéno, alkyle, alcényle, hydroxy, alcoxy, alcanoyle ou amido, et lesdits groupes alkyle, alcényle, alcoxy, alcanoyle ou amido étant éventuellement substitués par un ou plusieurs groupes halogéno ; et
"aralkyle" désigne un groupe alkyle tel que défini ci-dessus, substitué par un groupe aryle tel que défini ci-dessus.

2. Composition comprenant une huile lubrifiante et de 0,1 à 20 % d'un composé selon la revendication 1.

3. Composition selon la revendication 2, qui contient de 0,5 à 10 % d'un composé selon la revendication 1.

4. Utilisation d'un composé selon la revendication 1 en une quantité de 0,1 à 20 % pour améliorer les caractéristiques anti-usure et anti-corrosion d'une huile lubrifiante.
